Europäisches Patentamt

(19) **European Patent Office**

**Office européen des brevets**

(11) Publication number: **0 000 988**
**B1**

(12) **EUROPEAN PATENT SPECIFICATION**

(45) Date of publication of patent specification: **26.05.82**

(51) Int. Cl.³: **A 61 N 1/36**

(21) Application number: **78300242.1**

(22) Date of filing: **03.08.78**

(54) Demand cardiac stimulating apparatus.

(30) Priority: **19.08.77 GB 3491477**
**19.06.78 US 917129**

(43) Date of publication of application:
**07.03.79 Bulletin 79/5**

(45) Publication of the grant of the patent:
**26.05.82 Bulletin 82/21**

(84) Designated Contracting States:
**DE FR GB NL SE**

(56) References cited:
**FR - A - 2 329 007**
**LU - A - 76 880**
**US - A - 3 557 796**

(73) Proprietor: **BIOTRONIK Mess- und Therapiegeräte**
**GmbH & Co Ingenieurbüro Berlin**
**Sieversufer 8**
**D-1000 Berlin 47 (DE)**

(72) Inventor: **Digby, Dennis**
**2409 Gunflint Trail**
**Brooklyn Park Minnesota 55444 (US)**

(74) Representative: **Christiansen, Henning, Dipl.-Ing.**
**Unter den Eichen 108a**
**D-1000 Berlin 45 (DE)**

Courier Press, Leamington Spa, England.

Demand cardiac stimulating apparatus

Technical field

This invention relates to implantable body function control apparatus and particularly, to body tissue stimulating devices in the form of cardiac pacemakers.

Background art

Pacemakers for generating artificial stimulating pulses for the heart, and which may or may not be implanted in the body, are well-known. Pacemakers can be classified into demand and non-demand types. A demand pacemaker only issues an artificial pulse if the heart does not produce its own satisfactory natural beat, whereas a non-demand pacemaker issues artifical stimulating pulses without regard to the presence or absence of a natural beat.

A demand pacemaker normally includes an input amplifier for receiving and amplifying electrical signals from the heart (which signals might result from either a natural beat or an artificial pulse which has just been generated by the pacemaker), a pacemaker control circuitry which receives the amplified signals and which causes a new artificial stimulating pulse to be generated (for transmission to the heart) only if the amplified signals, or lack thereof, show that an artifical stimulating pulse is required by the heart (i.e. on demand), and an output amplifier which receives and amplifies the artifical pulses generated by the control circuitry, for passage to the heart.

Many types of pacemaker control circuitry as described above are available. Some function on an analog basis to produce the accurately-timed artifical stimulating pulses, whereas several recent designs employ digital circuitry.

With demand pacemakers, it is highly desirable to provide protection from the input circuitry from noise. Should a person bearing an implanted demand pacemaker meet an environment of high electrical noise, this noise may be picked up by the input amplifier which may then be incapable of distinguishing the picked-up signals from the signals it normally receives from the heart. In such circumstances, the circuitry is likely to consider the noise signals in the same manner as if it had received signals arising from a natural heart beat,—it will operate in its usual demand mode by not issuing artifical stimulating pulses to the heart. Since the heart may not be operating normally, and may indeed require artifical stimulation, these noise signals preventing such stimulation can lead to a dangerous condition for the person concerned.

Noise protection circuits have been incorporated into demand pacemakers in the past. For example, several include filters to attenuate noise signals of particular frequencies (particularly the commercial power mains frequency).

For example, one cardiac pacer affording a form of noise protection is known from French Patent Publication No. 2,329,007. This discloses a cardiac pacer wherein certain parameters including the refractory period and the mode of operation (fixed or demand) are controllable from an external circuit. Protection is afforded against adjustment of the pacer by noise signals. However, this protection only extends to noise signals received within the refractory period. If a disturbance signal with pulse intervals below a predetermined minimum interval commences after the end of the refractory period this is liable to erroneous evaluation as a signal arising due to the unaided action of the heart.

U.S. Patent No. 3,557,796 discloses a cardiac pacer in which a counter driven by an oscillator can be reset under certain conditions by ventricular or atrial input signals. Noise signals are detected before the period during which the pacer is sensitive to said input signals and, if such noise signals are detected, bring a noise protection circuit into operation. However, if a disturbance signal is initiated only very shortly before the sensitive period of the pacer, the number of noise pulses required for triggering the noise protection circuit is not attained.

Disclosure of invention

We have now developed a novel noise protection circuit for use in protecting demand pacemakers and which can be efficiently constructed with a small number of components of digital function. This is particularly useful in that it enables the components to be included in an integrated pacemaker circuit, e.g. of MSI or LSI construction.

According to the invention there is provided a demand cardiac stimulating apparatus comprising:

(a) oscillator means (1),

(b) first counter means (2), responsive to said oscillator means, for defining respectively increasing predetermined first, second and third count intervals, said first counter means being reset upon each achievement of said third count interval thereby to provide operation in a fixed rate mode, and being resettable independently of said counts at any time completely to reinitiate its full counting sequence thereby to provide operation in a demand mode;

(c) output amplifier means (3) for generating a stimulating pulse upon each achievement by said first counter means of said third count interval (CK3);

(d) input amplifier means (8) for sensing signals at the heart;

characterised by:

(e) second counter means (9), controllably incrementable upon each repetition by said first counter means of said first count interval (CK1), said second counter means being reset to recommence counting by each signal sensed at the heart by said input amplifier means;

(f) first control means (13), responsive to first achievement by said first counter means, after resetting thereof, of said second count interval (CK2), said first control means establishing a refractory period for said stimulating apparatus after each resetting of said first counter means and otherwise independently of the state of said second counter means; and

(g) gating means (10), responsive to a predetermined count of said second counter means, and second control means (11) inhibited by said first control means during said refractory period and thereafter being energized by said gating means for resetting said first counter means and thereby providing suppression of demand stimulation in response to sensed signals having periodicity of less than the interval defined by said predetermined count of said second counter means, and causing reversion to operation in the fixed rate mode.

Brief description of the drawings

Preferred features of the invention are illustrated in the accompanying drawings, in which:

Figure 1 shows a schematic electrical circuit diagram of an implantable, demand fixed-rate cardiac pacemaker according to the invention, and

Figure 2 is a timing diagram for use with Figure 1.

Best mode of carrying out the invention

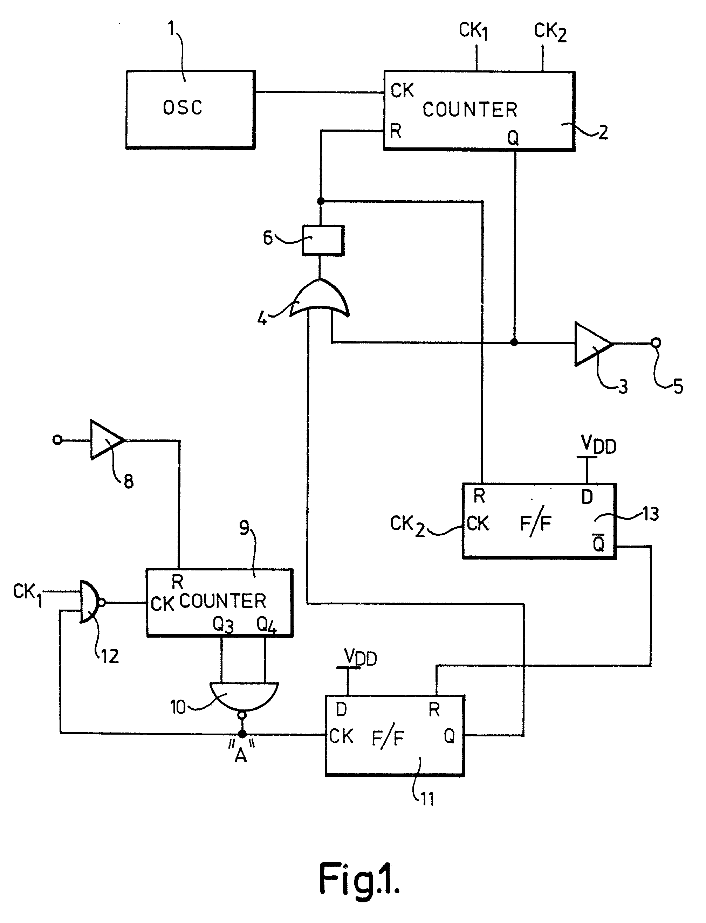

Referring to Figure 1, the pacemaker comprises an oscillator 1 which clocks a ripple counter 2. A cardiac stimulating pulse rate is obtained from an approriate output stage, Q, of counter 2 and is provided to an output amplifier 3 and to one input of an OR gate 4. The output amplifier 3 provides amplified tissue stimulating pulses to a connection 5 for coupling to an electrode leading to the heart. The output of OR gate 4 is supplied to a monostable 6, which is employed to reset counter 2.

The pacemaker also includes an input amplifier 8 which receives electrical signals generated at the heart (e.g. arising from natural heart beats) and supplies these signals, amplified, to the reset input of a ripple counter 9.

Two of the output stages of counter 9, nominated Q3 and Q4 provide inputs to a NAND gate 10, the output of which supplies the clock input to a D-type flip-flop 11 and an input to a NAND gate 12. The latter clocks ripple counter 9.

The Q output of flip-flop 11 supplies a second input to OR gate 4, and the D-input of flip-flop 11 is tied to the positive supply rail.

A further D-type flip-flop 13 is provided which is reset from the output monostable 6 and whose $\overline{Q}$ output is employed to reset flip-flop 11. The D-input of flip-flop 13 is tied to the positive supply rail.

The counter 2 also supplies two clock outputs, $CK_1$ and $CK_2$. $CK_1$, having a period of 8 msecs, is employed to provide a second input to NAND gate 12, and $CK_2$ is employed to clock flip-flop 13. The clock pulse $CK_2$ arises 320 msecs after counter 2 is reset.

The pacemaker operates as follows. In its fixed rate mode, with neither noise nor natural heart beats being detected and amplified by input amplifier 8, counter 2 issues a continuous series of tissue stimulating pulses to output amplifier 3. The reset for counter 2 after each pulse occurs via OR gate 4, and monostable 6. The counter 2 is reset on the trailing edge of the monostable pulse and the firing time of the latter thus determines the pulse width of each stimulating pulse issued by counter 2.

Each reset pulse provided by monostable 6 also resets flip-flop 13, holding its $\overline{Q}$ output high. This reset holds until flip-flop 13 is clocked by a clock pulse $CK_2$. Until this clocking occurs, the $\overline{Q}$ output of flip-flop 13 holds a reset on flip-flop 11. The purpose of flip-flop 13 is to create a refractory period of 320 msecs—a period of time after a pacemaker pulse or a natural beat during which any input to the pacemaker via input amplfier 8 has no effect on the pacemaker behavior.

The purpose of counter 9, flip-flop 11 and associated circuitry is to allow a single pulse at the input amplifier 8 to reset counter 2 if the pulse occurs outside the refractory period. In such a circumstance, the pacemaker acts in a normal demand mode—only issuing pacemaker pulses if a natural beat is missing.

For explanation of the normal demand mode operation, assume initially that counter 2 has issued a pulse to cause itself to reset via OR gate 4. This reset will have reset flip-flop 13 and this, via the $\overline{Q}$ output of the latter, will also reset flip-flop 11. The Q output of flip-flop 11 will be low. Assume also that counter 9 is full, in the sense that the Q3, Q4 stages are high. This will cause point "A" to be low, and the output of NAND gate 12 to be high, thus locking out further $CK_1$ clock pulses to counter 9.

Counter 2 recommences counting and until the count $CK_2$ is reached, the reset on flip-flop 11 will be held. On reaching $CK_2$, flip-flop 13 clocks and its $\overline{Q}$ output goes low, removing the flip-flop 11 reset. This is the end of the refractory period. Since counter 9 is locked out, and point "A" is locked low, flip-flop 11 is not clocked and its Q output remains low.

If a single input pulse is received by amplifier 8 after the end of the refractory period (i.e. as a result of a natural heart beat), counter 9 is reset, Q3 and Q4 go low, and the point "A" goes high to clock flip-flop 11. The Q output of the latter resets counter 2 via OR gate 4 to inhibit the pacemaker pulse being generated, and recommences the refractory period by resetting flip-flop 13. Counter 9, in the meantime, commences counting clock pulses $CK_1$ and locks out again when Q3, Q4 go high. It is of no consequence if the reset to counter 2 initiated by a natural heart beat arrives just as an artifical pulse is generated, since the natural beat and the stimulating pulse will essentially coincide.

If a series of input pulses are received having an interpulse period less than 88 msecs, counter 9 is continuously reset and, apart from the initial reset on the first of these pulses, point "A" does not make the low to high transition necessary to clock flip-flop 11. When this occurs, flip-flop 11 is then incapable of continuously resetting counter and the pacemaker reverts to the fixed rate mode described above. The effect of the noise protection circuitry is hence to cause the pacemaker to operate in its fixed rate, non-demand mode in the presence of noise (or heart generated signals) having a period less than 88 msec. This figure of 88 msec arises in that it is the minimum time required to fill counter 9 (i.e. Q3 and Q4 high) after a first input pulse is received via amplifier 8 to reset counter 9. The maximum time required to fill counter 9 is 96 msec. The spread of 88 to 96 msec (1 clock period) is due to the asynchronous nature of the incoming signal to amplifier 8 compared to the next occurrence of a $CK_1$ clock pulse.

If a single input pulse is received during the last 89—96 msec of the refractory period, this effectively acts to extend the refractory period by up to 96 msec maximum, to provide a "system refractory period" of 416 msec maximum. This "refractory period extension" may be beneficial to the pacemaker in that extraneous signals present towards the latter part of the refractory period are ignored (as being natural heart beats) and the chance of the pacemaker being inhibited by unwanted signals (e.g. a large T-wave from the heart) is reduced.

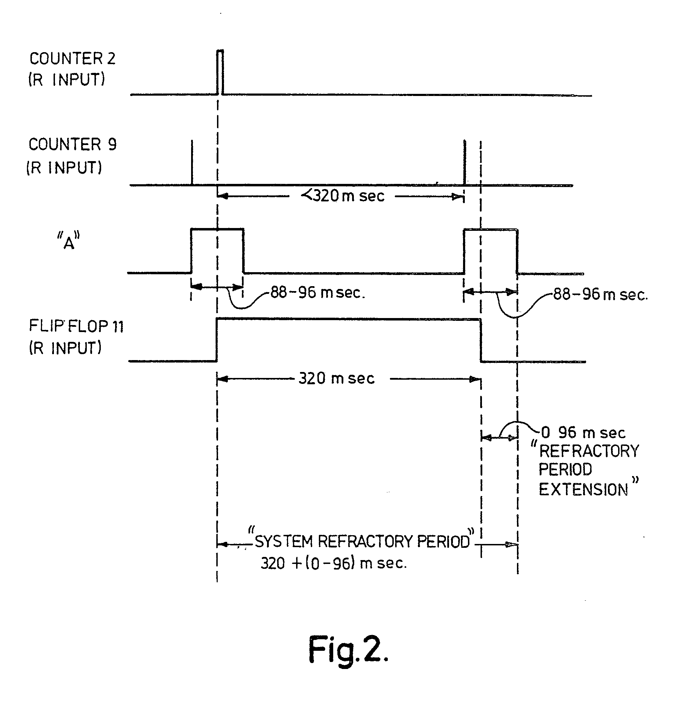

This phenomenon of "refractory period extension" is best understood with reference to the timing diagram of Figure 2. A reset to counter 9 arises towards the end of the refractory period and this sends the point "A" (clock input to flip-flop 11) high. The period for which "A" remains high after the refractory period of 320 msec expires (a maximum of 96 msecs) acts as a "refractory period extension". Any input to amplifier 8 causing a reset on counter 9 during this extended period (the "system refractory period") will not inhibit the count being generated by the counter 2.

## Claims

1. Demand cardiac stimulating apparatus comprising:
   (a) oscillator means (1);
   (b) first counter means (2), responsive to said oscillator means, for defining respectively increasing predetermined first, second and third counter intervals, said first counter means being reset upon each achievement of said third counter interval thereby to provide operation in a fixed rate mode, and being resettable independently of said counts at any time completely to reinitiate its full counting sequence thereby to provide operation in a demand mode.
   (c) output amplifier means (3) for generating a stimulating pulse upon each achievement by said first counter means of said third count interval (CK3);
   (d) input amplifier means (8) for sensing signals at the heart;
   characterised by:
   (e) second counter means (9), controllably incrementable upon each repetition by said first counter means of said first counter interval (CK1), said second counter means being reset to recommence counting by each signal sensed at the heart by said input amplifier means;
   (f) first control means (13), responsive to first achievement by said first counter means, after resetting thereof, of said second count interval (CK2), said first control means establishing a refractory period for said stimulating apparatus after each resetting of said first counter means and otherwise independently of the state of said second counter means; and
   (g) gating means (10), responsive to a predetermined count of said second counter means and second control means (11) inhibited by said first control means during said refractory period and thereafter being energized by said gating means for resetting said first counter means and thereby providing suppression of demand stimulation in response to sensed signals having periodicity of less than the interval defined by said predetermined count of said second counter means, and causing reversion to operation in the fixed rate mode.

2. An apparatus according to claim 1 characterised in that said first control means (13) comprises a D-type flip-flop having its D-input maintained at a logic 1 state, its reset input (R) connected to the reset input (R) of said first counter means (2), its clock input (CK) controlled by said second count interval (CK2) of said first counter means (2), and its $\overline{Q}$ output providing control for said second control means (11).

3. An apparatus according to claim 2 characterized in that said second control means (11) comprises a D-type flip-flop having its D-input maintained at a logic 1 state, its reset input (R) coupled to said $\overline{Q}$ output of said first control means (13), its clock input (CK) controlled by said gating means (10), and its Q output coupled to reset said first counter means (2).

## Patentansprüche

1. Bedarfs-Herzschrittmacher, bestehend aus:
   a) Oszillatormitteln (1);

b) ersten Zählmitteln (2), welche auf die Oszillatormittel ansprechen, um vorbestimmte zunehmende erste, zweite und dritte Zählintervalle festzulegen, wobei die Zählmittel bei jedem Erreichen des dritten Zählintervalls zurückgesetzt werden, auf diese Weise einen Betrieb mit fester Rate einschalten und unabhängig von den Zählintervallen jederzeit vollständig rücksetzbar sind, um ihre volle Zählsequenz einzuleiten, womit der Bedarfs-Betriebszustand verbunden ist;

c) Ausgangsverstärkermitteln (3) zur Erzeugung eines Stimulationsimpulses bei jedem Erreichen des dritten Zählintervalls (CK3) durch die ersten Zählmittel;

d) Eingangsverstärkermitteln (8) zum Aufnehmen von Signalen am Herzen, gekennzeichnet durch:

e) zweite Zählmittel (9), welche bei jeder Wiederholung des ersten Zählintervalls (CK1) durch die ersten Zählmittel gesteuert heraufsetzbar sind, wobei die zweiten Zählmittel zurückgesetzt werden, um bei jedem durch die Eingangsverstärkermittel vom Herzen aufgenommenen Signal erneut mit dem Zählen zu beginnen;

f) erste Zählmittel (13), welches beim ersten Erreichen des zweiten Zählintervalls (CK2) durch die ersten Zählmittel nach deren Zurücksetzen ansprechen, wobei die ersten Steuermittel eine Refraktärzeit für die Stimulationsvorrichtung unabhängig vom Zustand der zweiten Zählmittel, jedesmal wenn die ersten Zählmittel zurückgesetzt wurden, erzeugen, und

g) Tormittel (10), welche auf den vorgegebenen Zählerstand der zweiten Zählmittel ansprechen sowie zweite Steuermittel (11), welche durch die ersten Steuermittel während der Refraktärzeit inhibiert werden und daraufhin durch die Tormittle zum Zurücksetzen der ersten Zählmittel aktiviert werden, wodurch die Bedarfsstimulation auf die aufgenommenen Signale mit einer Periodizität, die kleiner ist als der vorgegebene Zählerstand des zweiten Zählers, unterdrückt wird und eine Rückkehr in den Betriebszustand mit fester Rate erfolgt.

2. Vorrichtung nach Anspruch 1, dadurch gekennzeichnet, daß die ersten Steuermittel (13) ein D-Flip-Flop aufweisen, wobei der D-Eingang auf dem logischen 1-Zustand gehalten wird, der Rücksetz-Eingang (R) mit dem Rücksetz-Eingang (R) der ersten Zählmittel (2) verbunden ist, sine Takt-Eingang (CK) durch das zweite Zählintervall (CK2) der ersten Zählmittel (2) gesteuert wird und sein Q-Ausgang die zweiten Steuermittel (11) ansteuert.

3. Vorrichtung nach Anspruch 2, dadurch gekennzeichnet, daß die zweiten Steuermittel (11) ein D-Flip-Flop aufweisen, dessen D-Eingang auf logischem 1-Zustand gehalten wird, wobei der Rücksetz-Eingang (R) mit dem Q̄-Ausgang der ersten Steuermittel (13) verbunden ist, während sein Takt-Eingang (CK) durch die Tormittel (10) gesteuert und sein Q-Ausgang mit den Zählmitteln (2) für deren Rücksetzen verbunden ist.

**Revendications**

1. Appareil de stimulation cardiaque à intervention en cas nécessité, comprenant:

(a) un moyen oscillateur (1);

(b) un premier moyen de comptage (2), répondant audit moyen oscillateur, de façon à définir un premier, un deuxième et un troisième intervalle de comptage prédéterminés respectivement croissants, ledit premier moyen de comptage étant repositionné à chaque achèvement dudit troisième intervalle de comptage de manière à produire un fonctionnement dans un mode à rythme fixe, et étant repositionnable indépendamment desdits comptages à tout instant de manière totale pour réinitialiser sa séquence de comptage complète de manière à produire un fonctionnement dans un mode à intervention en cas de nécessité;

(c) un moyen amplificateur de sortie (3) destiné à produire une impulsion de stimulation à chaque achèvement, par ledit premier moyen de comptage, dudit troisième intervalle de comptage (CK3);

(d) un moyen amplificateur d'entrée (8) destiné à détecter des signaux au niveau du coeur;

caractérisé par:

(e) un deuxième moyen de comptage (9) qui peut être incrémenté de manière réglable à chaque répétition, par ledit premier moyen de comptage, dudit premier intervalle de comptage (CK1), ledit deuxième moyen de comptage étant repositionné afin de recommencer à compter par chaque signal détecté au niveau du coeur par ledit moyen amplificateur d'entrée;

(f) un premier moyen de commande (13), répondant à un premier achèvement, par ledit premier moyen de comptage, après son repositionnement, dudit deuxième intervalle de comptage (CK2), ledit premier moyen de commande établissant une période réfractaire pour ledit appareil de stimulation après chaque repositionnement dudit premier moyen de comptage et, dans les autres cas, indépendamment de l'état dudit deuxième moyen de comptage; et

(g) un moyen de déclenchement (10), répondant à un comptage prédéterminé dudit deuxième moyen de comptage, et un deuxième moyen de commande (11) neutralisé par ledit premier moyen de commande pendant ladite période réfractaire et étant, après cela, excité par ledit moyen de déclenchement de manière à repositionner ledit premier moyen de comptage et, ainsi, permettre la suppression de la stimulation intervenant en cas de nécessité en réponse à des signaux détectés ayant une périodicité moindre que l'intervalle défini par ledit comptage prédéterminé dudit deuxième moyen de comptage, et à provoquer le retour au fonctionnement dans le mode à rythme fixe.

2. Appareil selon la revendication 1, caractérisé en ce que ledit premier moyen de commande (13) comprend une bascule de type D dont l'entrée D est maintenue à un état logique 1, dont l'entrée de repositionnement (R) est connectée à l'entrée de repositionnement (R) dudit premier moyen de comptage (2), dont l'entrée de cadencement (CK) est commandée par ledit deuxième intervalle de comptage (CK2) dudit premier moyen de comptage (2), et dont la sortie Q̄ assure la commande dudit deuxième moyen de commande (11).

3. Appareil selon la revendication 2, caractérisé en ce que ledit deuxième moyen de commande (11) comprend une bascule de type D dont l'entrée D est maintenue à un état logique 1, dont l'entrée de repositionnement (R) est couplée à ladite sortie Q̄ dudit premier moyen de commande (13), dont l'entrée de cadencement (CK) est commandée par ledit moyen de déclenchement (10), et dont la sortie Q est couplée de façon à repositionner ledit premier moyen de comptage (2).

**0 000 988**

Fig.1.

1

Fig.2.